# EUROPÄISCHE PATENTANMELDUNG

(11) **EP 2 578 145 A1**
(43) Veröffentlichungstag der Anmeldung: **10.04.2013**
(21) Anmeldenummer: 13150166.0
(22) Anmeldetag: 11.09.2008
(51) Int. Cl.: A61B 5/03, A61B 5/00

(54) **Verfahren zur Übertragung von Sensordaten eines implantierbaren Hirnparametersensors auf ein externes Datenlesemodul**

(30) Priorität: 28.09.2007 DE 102007046694
(62) Teilanmeldung aus: 08802003.7
(71) Anmelder: RAUMEDIC AG, 95213 Münchberg (DE)
(72) Erfinder: Tauber, Karsten, Dr., 39110 Magdeburg (DE); von Falkenhausen, Christian, Dr., 53340 Meckenheim (DE); Kunze, Hans Gerd, 08297 Zwönitz (DE); Göhler, Karlheinz, 08297 Zwönitz (DE)
(74) Vertreter: Rau, Schneck & Hübner

(57) **Zusammenfassung**

Ein Verfahren zur Übertragung von Sensordaten eines implantierbaren Hirnparametersensors auf ein externes Datenlesemodul umfasst die Verfahrensschritte Senden eines von einer Hochfrequenzquelle des Datenlesemoduls erzeugten Hochfrequenz-Signals, Empfangen des Hochfrequenz-Signals mit einer Sendeeinheit (4) des Hirnparametersensors, Gleichrichten des Hochfrequenz-Signals, Aufladen des Kondensators zur zeitweiligen Energieversorgung der Sendeeinheit (4), Deaktivieren des Hochfrequenz-Signals nach dem Ladevorgang, Aufnehmen eines Hirnparameter-Messsignals, insbesondere eines Hirndruckwertes, wobei während des Aufnehmens des Hirnparameter-Messsignals eine Energieversorgung des Hirnparametersensors über den Kondensator (6) erfolgt, Aktivieren des Hochfrequenz-Signals und Senden des Hirnparameter-Messsignals mit dem Hochfrequenzsignal als Trägerfrequenz mittels der Sendeeinheit (4) an eine Empfangseinheit (8) des Datenlesemoduls.

## Beschreibung

Die Erfindung betrifft ein Verfahren zur Übertragung von Sensordaten eines implantierbaren Hirnparametersensors auf ein externes Datenlesemodul.

Ein Sensorsystem ist bekannt aus der W02004/023993 A1. Weitere Sensorsysteme sind bekannt aus der EP 1 312 302 A2, der DE 696 34 810 T2, der DE 197 05 474 A1, der DE 696 34 689 T2, der US 4,519,401 A und der US 2003/0023146 A1.

Aus der DE 102 15 115 A1 ist eine EEG-Kappe mit Sensoren bekannt, die zur Ausbildung einer Messeinrichtung in einer Vorrichtung zur reaktiven automatischen nicht invasiven gesteuerten bzw. geregelten elektromagnetischen Prävention epileptischer Anfälle in vivo ein Sensorgitter bilden.

Hirnparametermessungen finden insbesondere mit dem Ziel statt, die Notwendigkeit für spätere Therapiemaßnahmen abzuklären. Ein Anwendungsbeispiel ist hierfür eine Hirndruck-Langzeitmessung zur Klärung der Frage, ob ein Hydrocephaluspatient ein Shuntventil benötigt und wie ein derartiges Ventil hinsichtlich seiner Dimensionierung ausgelegt werden muss. Hierfür ist eine Datenerfassung des Hirndruckverlaufes über einen längeren Zeitraum, beispielsweise über Nacht oder über mehrere Tage erforderlich, um aus der Dynamik des Hirndrucks (ICP, Intracranial Pressure) eine entsprechende Diagnose ableiten zu können.

Der Einsatz der bisher bekannten Sensorsysteme zur Bearbeitung derartiger Fragestellungen ist für den Patienten sehr unbequem und beschwerlich.

Es ist eine Aufgabe der vorliegenden Erfindung, ein Verfahren zur Übertragung von Sensordaten eines implantierbaren Hirnparametersensors auf ein externes Datenlesemodul zu verbessern.

Diese Aufgabe wird erfindungsgemäß gelöst durch ein Verfahren mit den im Anspruch 1 angegebenen Merkmalen.

Erfindungsgemäß wurde erkannt, dass ein Verfahren zur Übertragung von Sensordaten eines implantierbaren Hirnparametersensors auf ein externes Datenmodul dann verbessert ist, wenn es die folgenden Schritte aufweist: Senden eines von einer Hochfrequenzquelle des Datenlesemoduls erzeugten Hochfrequenz-Signals, Empfangen des Hochfrequenz-Signals mit einer Sendeeinheit des Hirnparametersensors, Gleichrichten des Hochfrequenz-Signals, Aufladen eines Kondensators zur zeitweiligen Energieversorgung der Sendeeinheit, Deaktivieren des Hochfrequenz-Signals nach dem Ladevorgang, Aufnehmen eines Hirnparameter-Messsignals, insbesondere eines Hirndruckwertes, wobei während des Aufnehmens des Hirnparameter-Messsignals eine Energieversorgung des Hirnparametersensors über den Kondensator erfolgt, Aktivieren des Hochfrequenz-Signals, Senden des Hirnparameter-Messsignals mit dem Hochfrequenz-Signal als Trägerfrequenz mittels der Sendeeinheit an eine Empfangseinheit des Datenlesemoduls.

Weitere vorteilhafte Ausgestaltungen der Erfindung ergeben sich aus den Unteransprüchen.

Ein Sensorsystem zur Messung, Übertragung, Verarbeitung und Darstellung eines Hirnparameters mit mindestens einem implantierbaren Hirnparametersensor mit einer drahtlosen Sendeeinheit zur Messung des Hirnparameters, mit mindestens einer Empfangseinheit mit einer Antenne, die mit der Sendeeinheit in drahtloser Signalverbindung steht, mit mindestens einem Datenlesemodul, welches mit der Antenne über eine Signalverbindung in Signalverbindung steht, und mit mindestens einer Datenverarbeitungs- und -darstellungseinrichtung, welche mit dem Datenlesemodul in Signalverbindung steht, wobei eine Kopfhaube, an der die Empfangseinheit zur Vorgabe einer Relativpositionierung relativ zur Sendeeinheit festgelegt ist, vorgesehen ist, ermöglicht, dass diejenigen Komponenten, die zum Empfangen der Messdaten vom implantierten Hirnparametersensor erforderlich sind, so leicht und bauklein gestaltet werden können, dass sie ohne weiteres an einer Kopfhaube befestigt werden können. Die Kopfhaube schützt die Empfangseinheit. Der Patient kann sich mit der Kopfhaube im Wesentlichen frei bewegen, muss also während der Messdauer nicht stationär untergebracht sein. Die Kopfhaube stellt eine exakte Relativposition der Empfangseinheit zur Sendeeinheit sicher.

Eine Antenne, die Teil einer gedruckten Schaltung ist, ist besonders leicht und bauklein und damit komfortabel zu tragen.

Eine Klettverbindung, mittels der die Empfangseinheit an der Kopfhaube festgelegt ist, erlaubt eine ortsflexible Relativpositionierung zur Justierung der Empfangseinheit relativ zur Sendeeinheit. Hierzu kann die Empfangseinheit gegenüber der Kopfhaube stufenlos verlagert werden, bis eine gewünschte Soll-Signalstärke bei der Übertragung zwischen der Sendeeinheit und der Empfangseinheit erreicht ist. Insbesondere ist es möglich, Symmetrieachsen der Sendeeinheit und der Empfangseinheit miteinander praktisch vollständig zur Überdeckung zu bringen, was eine sehr gute Qualität der Signalübertragung gewährleistet.

Eine Gitternetz-Kopfhaube hat sich beim Einsatz im Zusammenhang mit EEG-(Elektroenzephalographie-)Systemen bewährt. Eine entsprechende Gitternetz-Kopfhaube kann auch, nach Ausrüstung mit einer entsprechenden Empfangseinheit, beim Sensorsystem mit einer Kopfhaube, die eine Mehrzahl von Einzelbändern oder -schläuchen aufweist, die ein Gitternetz vorgeben, zum Einsatz kommen, wobei die Empfangseinheit an Kreuzungspunkten des Gitternetzes festgelegt ist.

Ein Gehäusering, über den die Empfangseinheit an einem Patientenkopf anlegbar ist, gewährleistet eine sichere Anlage der Empfangseinheit am Patientenkopf. Da die Sendeeinheit in der Regel zwischen der Kopfhaut und der Kalotte untergebracht ist, stellt die Sendeeinheit eine deutlich fühlbare Erhebung am Patientenkopf dar. Der Gehäusering ist bevorzugt so dimensioniert, dass er diese Erhebung zumindest abschnittsweise umschließt, was die Relativpositionierung der Sendeeinheit zur Empfangseinheit verbessert.

In dem am Patientenkopf anlegbaren Gehäuse bzw. Gehäusering kann nur ein Teil der die Empfangseinheit aufbauenden Komponenten untergebracht sein. Bevorzugt ist lediglich die Antenne der Empfangseinheit im Gehäuse bzw. Gehäusering untergebracht, wohingegen die anderen Komponenten der Empfangseinheit beispielsweise über eine kabelgebundene oder auch über eine kabellose Signalverbindung mit der Antenne in Verbindung stehen. Die am Patientenkopf anlegbare Komponente kann dann leicht und kompakt ausgestaltet sein.

Ausgestaltungen mit einer flexiblen Membran, über die die Empfangseinheit an dem Patientenkopf anlegbar ist, wobei zwischen der flexiblen Membran und der Antenne eine formveränderliche Schicht, insbesondere eine Gelschicht, angeordnet sein kann, verbessern die Anlage der Empfangseinheit an der Sendeeinheit und damit die Relativpositionierung dieser beiden Einheiten zueinander.

Eine Integration des Datenlesemoduls und der Empfangseinheit in eine integrierte Baueinheit, die an der Kopfhaube festlegbar ist, vermeidet insbesondere, dass der Patient neben der Kopfhaube noch zusätzliche Komponenten beispielsweise am Gürtel tragen muss.

Die Vorteile einer Kopfhaube mit einer Empfangseinheit zum Einsatz in einem Sensorsystem entsprechen denen, die vorstehend unter Bezugnahme auf das Sensorsystem bereits ausgeführt wurden.

Ein Ausführungsbeispiel der Erfindung wird nachfolgend anhand der Zeichnung näher erläutert. In dieser zeigen:
- Fig. 1: ein Sensorsystem zur Messung, Übertragung, Verarbeitung und Darstellung eines Hirnparameters;
- Fig. 2: schematisch einen Schnitt durch eine auf einen Patientenkopf aufgesetzte Kopfhaube des Sensorsystems im Bereich einer an der Kopfhaube festgelegten Antenne.

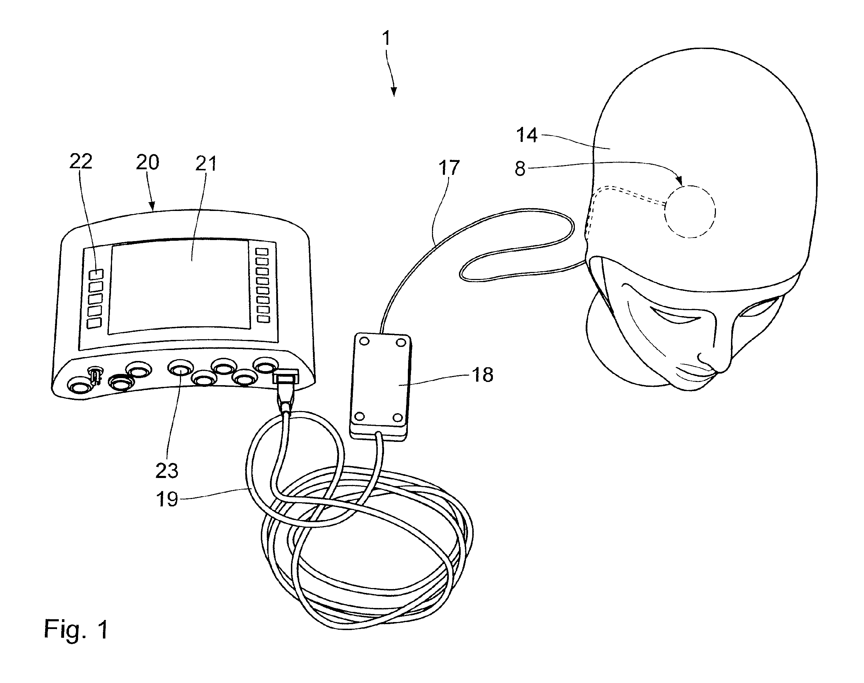

Ein in der Fig. 1 insgesamt dargestelltes Sensorsystem 1 dient zur Messung, Übertragung, Verarbeitung und Darstellung eines Hirnparameters, insbesondere eines Hirndrucks.

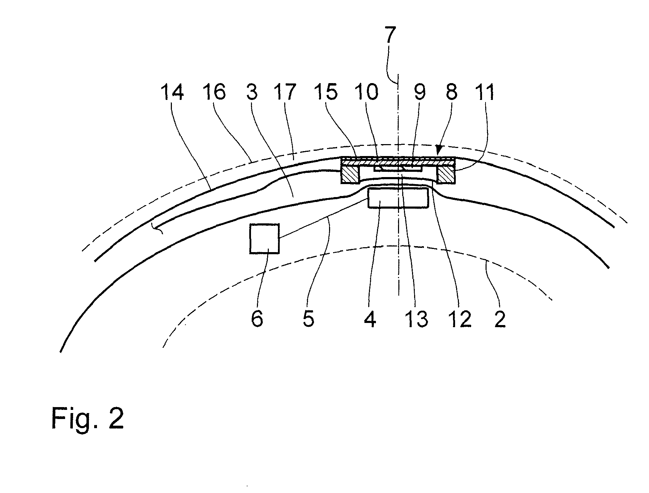

Zum Sensorsystem 1 gehört ein nicht näher dargestellter, in einen Patientenkopf implantierbarer Hirnparametersensor. Der ausschnittsweise Querschnitt der Fig. 2 zeigt Komponenten des implantierten Hirnparametersensors, die zwischen einer gestrichelt in der Fig. 2 angedeuteten Kalotte 2 und der Kopfhaut 3 eines Patienten angeordnet sind. Hierzu gehört ein drahtloser Sensortransponder 4 mit einer Antennenspule. Der Sensortransponder 4 wird nachfolgend auch als Sendeeinheit bezeichnet. Die Sendeeinheit 4 steht über eine Leitung 5 sowie eine nicht dargestellte Gleichrichtung mit einem Kondensator 6 in elektrischem Kontakt steht. Die Darstellung nach Fig. 2 ist äußerst schematisch und nicht maßstabsgerecht.

Der Sensortransponder 4 hat keine eigene Energiequelle. Der Sensortransponder 4 weist zusätzlich einen nicht näher dargestellten A/D-Wandler auf. Dieser dient zur Digitalisierung der vom Sensortransponder 4 weiterzugebenden Sensor-Messdaten.

Eine Rotationssymmetrieachse 7 der Antennenspule der Sendeeinheit 4 ist in der Fig. 2 gestrichelt angedeutet. Die Achse 7 verläuft bedingt durch die Anordnung der Sendeeinheit 4 im Wesentlichen normal zur Außenwand der Kalotte 2 im Bereich der Auflage der Antennenspule der Sendeeinheit 4 auf dieser.

Über eine nicht dargestellte Signalverbindung ist die Sendeeinheit 4 mit einem Sensorelement des Hirnparametersensors verbunden. Das Sensorelement ist beispielsweise als durch die Kalotte 2 hindurch geführter Katheter mit einem Druckmesskopf ausgeführt.

Der Sendeeinheit 4 zugeordnet ist eine äußere Sende/Empfangseinheit 8 des Sensorsystems 1, die nachfolgend auch nur als Empfangseinheit oder als Reader bezeichnet wird. Die Empfangseinheit 8 ist außerhalb des Patientenkopfes angeordnet. Die Empfangseinheit 8 hat eine Antenne 9, die drahtlos zur Energieversorgung sowie zur Datenübermittlung mit der Sendeeinheit 4 in Signalverbindung steht. Die Antenne 9 ist als Teil einer gedruckten Schaltung (PCB, printed circuit board) auf einer dünnen Platine 10 mit einer Stärke zwischen 0,8 mm und 1,5 mm aufgedruckt. Auch eine noch dünnere Platine ist möglich. Zur Verbesserung der Anpassungsfähigkeit der Empfangseinheit 8 kann die Platine 10 als flexible, also als biegsame Platine ausgebildet sein. Die Antenne 9 hat ebenfalls die Form einer Spule, deren Rotationssymmetrieachse mit der Achse 7 zusammenfällt. Die Spulenwindungen der Einheiten 4, 8 können insbesondere spiralförmig angeordnet sein.

Die Platine 10 ist an einem ringförmigen Gehäuse 11 mit einem Durchmesser von einigen cm befestigt. Auch die Rotationssymmetrieachse des Gehäuses 11 fällt mit der Achse 7 zusammen. Der Befestigungsort der Platine 10 ist auf einer von der Kopfhaut 3 abgewandten Seite des Gehäuses 11.

Ferner ist am Gehäuse 11 an der der Kopfhaut 3 zugewandten Seite eine flexible Membran 12 festgelegt. Die Membran 12 liegt somit zwischen der Antenne 9 und der Kopfhaut 3.

Zwischen der Antenne 9 und der Membran 12 ist eine Schicht 13 aus einem nachgiebigen, formveränderlichen Material, insbesondere aus einem fließfähigen und hochviskosem Material, insbesondere aus einem Gel angeordnet.

Beim Auflegen der Empfangseinheit 8 über das Gehäuse 11 auf die Kopfhaut 3 im Bereich der Sendeeinheit 4 passt sich aufgrund einer gezielten Verdrängung der Schicht 13 die Membran 12 an die Form des Patientenkopfes an dieser Stelle an, so dass ein korrekter Sitz der Empfangseinheit 8 relativ zur Sendeeinheit 4 gewährleistet ist.

Die Empfangseinheit 8 ist an der Innenseite einer Kopfhaube 14 festgelegt, die ebenfalls Teil des Sensorsystems 1 ist. Zur Befestigung der Empfangseinheit 8 an der Kopfhaube 14 dient eine Klettverbindung mit einem Klettband 15, das auf der der Kopfhaube 14 zugewandten Seite auf das Gehäuse 11 und die Platine 10 der Empfangseinheit 8 aufgeklebt ist. Über diese Klettverbindung lässt sich die Empfangseinheit 8 an einer beliebigen Stelle auf der Innenseite der Kopfhaube 14 anbringen, so dass eine gewünschte Relativpositionierung der Empfangseinheit 8 zur Sendeeinheit 4 möglich ist. Die Kopfhaube 14 ist aus einem dehnbaren und eng am Patientenkopf anliegenden Gaze oder Baumwollmaterial. Die Kopfhaube 14 ist im Schnitt an die Form des Patientenkopfes angepasst. Die Kopfhaube 14 kann zur Fixierung am Patientenkopf ein in der Zeichnung nicht dargestelltes Kinnband aufweisen. Zusätzlich zur Kopfhaube 14 kann auch noch eine in Fig. 2 gestrichelt angedeutete äußere Überhaube 16 vorgesehen sein, die Kräfte von außen aufnimmt und somit die dann innere Kopfhaube 14 gegen ein unerwünschtes Verrutschen relativ zur Kalotte 2 sichert.

Über ein Signalkabel 17, welches abschnittsweise zwischen der Kopfhaut 3 und der Kopfhaube 14 verläuft, steht die Antenne 9 der Empfangseinheit 8 mit einem Datenlesemodul 18 in Signalverbindung. Das Datenlesemodul 18 umfasst eine Steuereinheit zur Steuerung des implantierten Hirnparametersensors über die Empfangseinheit 8, über die eine bidirektionale Datenübertragung möglich ist. Ferner hat das Datenlesemodul 18 eine Hochfrequenzquelle zur Erzeugung einer Trägerfrequenz von 13,56 MHz. Das Datenlesemodul 18 hat zudem einen Signalspeicher. Das Datenlesemodul 18 hat in etwa die Größe einer Zigarettenschachtel und kann vom Patienten am Gürtel getragen werden.

Über ein weiteres Signalkabel 19 steht das Datenlesemodul 18 mit einer Datenverarbeitungs- und -darstellungseinrichtung 20 in Signalverbindung. Letztere hat ein Display 21 sowie eine Mehrzahl von Bedienknöpfen 22 und Anschlussbuchsen 23, insbesondere mindestens eine USB-Schnittstelle. Die Datenverarbeitungs- und -darstellungseinrichtung 20 ist mit einer externen Spannungsquelle verbindbar. Alternativ oder zusätzlich kann die Datenverarbeitungs- und -darstellungseinrichtung 20 eine interne Energiequelle, beispielsweise in Form einer Batterie oder eines aufladbaren Akkumulators, aufweisen. Über diese Energiequelle werden das Datenlesemodul 18, die Empfangseinheit 8 und drahtlos während eines Messvorgangs auch die Sendeeinheit 4 versorgt.

Bei der Signalverbindung zwischen der Antenne 9 und dem Datenlesemodul 18 einerseits und dem Datenlesemodul 18 und der Datenverarbeitungs- und -darstellungseinrichtung 20 andererseits kann es sich um eine USB-Schnittstelle oder auch um eine RS232-Schnittstelle, also um ein digitales Interface, handeln.

Ein Hirnparameter-Messvorgang, der über einen längeren Zeitraum, beispielsweise im Verlauf mehrerer Tage, regelmäßig wiederholt wird, läuft folgendermaßen ab:
Das Datenlesemodul 18 sendet über die Empfangseinheit 8 zunächst ein HF-Signal an die Sendeeinheit 4. Das HF-Signal wird gleichgerichtet und lädt den Kondensator 6 zur zeitweiligen Energieversorgung der Sendeeinheit 4 auf. Nach diesem Ladevorgang wird HF-Signal deaktiviert. Anschließend nimmt der Hirnparametersensor, versorgt über den Kondensator 6, das Hirnparameter-Messsignal, beispielsweise einen Hirndruckwert, auf. Anschließend wird das HF-Signal wieder aktiviert und die Sendeeinheit 4
sendet mit dem HF-Signal als Trägerfrequenz den über den A/D-Wandler digitalisierten Messwert als digital moduliertes Signal an die Empfangseinheit 8. In der Empfangseinheit 8 erfolgt eine Überführung bzw. Dekodierung des modulierten HF-Signals in ein digitales, PC-kompatibles Signal. Je nach dem Schnittstellen-Standard, der beim Sensorsystem 1 eingesetzt wird, kann es sich beispielsweise um ein USB- oder um ein RS232-Signal handeln. Dieses überführte Messsignal wird an das Datenlesemodul 18 über das Signalkabel 17 übertragen. Das übertragene Signal wird dann über das Signalkabel 19 an die Datenverarbeitungs- und -darstellungseinrichtung 20 zur weiteren Verarbeitung und Darstellung übermittelt.

An Stelle einer Gaze- oder Baumwoll-Ausführung der Kopfhaube 14 und gegebenenfalls der Überhaube 16 kann die Kopfhaube 14 auch als Netz aus einer Mehrzahl von Einzelbändern oder -schläuchen ausgebildet sein. Durch diese Einzelbänder oder -schläuche wird dann ein Gitternetz vorgegeben. Die Empfangseinheit 8 kann dann Befestigungsmittel an den Einzelbändern oder -schläuchen aufweisen, die es insbesondere erlauben, die Empfangseinheit 8 an Kreuzungspunkten dieses Gitternetzes zu befestigen. Entsprechende Ausführungen von Kopfhauben aus Einzelbändern oder -schläuchen sind im Zusammenhang mit EEG-Kopfhauben bekannt.

Bei einer weiteren Variante des Sensorsystems 1 sind die Empfangseinheit 8 und das Datenlesemodul 18 als integrierte Baueinheit ausgeführt und an der Kopfhaube 14 befestigt. Zur Gewichtsverteilung dieser integrierten Baueinheit kann das Datenlesemodul auch von der Empfangseinheit 8 separat innen an der Kopfhaube 14 befestigt sein, beispielsweise an einer der Empfangseinheit 8 gegenüberliegenden Position. Die Empfangseinheit 8 und das Datenlesemodul 18 können dann über ein kurzes Signalkabel miteinander verbunden sein.

Eine entsprechende Integration der Empfangseinheit 8 ist auch mit Einzelkomponenten des Datenlesemoduls 18 möglich, beispielsweise mit der HF-Quelle oder mit der Steuereinheit des Datenlesemoduls 18. Die restlichen Komponenten sind dann wiederum über eine Signalverbindung mit einem externen, also außerhalb der Kopfhaube 14 angebrachten, Modul verbunden.

Bei diesen integrierten Varianten, bei denen die Empfangseinheit 8 noch zusätzliche Komponenten des Datenlesemoduls 18 aufweist, ist es auch möglich, dass die Empfangseinheit 8 eine eigene Energieversorgung, beispielsweise in Form einer Knopfzelle aufweist. In diesem Fall kann auf eine kabelgebundene Signalverbindung auch verzichtet werden und an Stele des Signalkabels 17 kann eine drahtlose Signalverbindung, beispielsweise eine Bluetooth-Signalverbindung zum Einsatz kommen.

Bei einer weiteren Variante des Sensorsystems 1 hat das Datenlesemodul 18 eine eigene Energieversorgung und an Stelle einer kabelgebundenen Signalverbindung mit der Datenverarbeitungs- und -darstellungseinrichtung 20 (Signalkabel 19) liegt eine drahtlose Signalverbindung vor, die beispielsweise ebenfalls über eine Bluetooth-Verbindung realisiert sein kann.

Die Datenverarbeitungs- und -darstellungseinrichtung 20 muss nicht ständig mit dem Datenlesemodul 18 in Signalverbindung stehen. Es reicht aus, wenn die Datenverarbeitungs- und -darstellungseinrichtung 20 die aufgenommenen Messdaten aus dem Datenlesemodul 18 nach Abschluss der beispielsweise mehrtätigen Messzeit ausliest. Erst dann ist eine entsprechende Datenverbindung erforderlich, so dass der Patient während der Messzeit nur die Kopfhaube 14 mit der Empfangseinheit 8 und das Datenlesemodul 18 tragen muss.

Die ortsflexible Positionierung der Empfangseinheit 8 über die Klettverbindung mit dem Klettband 15 ermöglicht eine exakte Ausrichtung der Empfangseinheit 8 zur Sendeeinheit 4, so dass die Spulenachsen dieser beiden Einheiten 4, 8 zusammenfallen. Dies gewährleistet eine optimale Signalübertragung zwischen den Einheiten 4 und 8.

Bei einer nicht näher dargestellten Ausführung ist die Empfangseinheit 8 mit einem ringförmigen Gehäuse 11 ausgeführt, in das eine ebenfalls ringförmige Antenne 9 eingearbeitet ist. Diese Ringantenne wird bei dieser Ausführungsform am Kopf des Patienten aufgesetzt. Die restliche Empfangseinheit, also die weiteren Komponenten, die bei der Ausführung nach den Figuren 1 und 2 insbesondere auf der gedruckten Schaltung auf der Platine 10 ausgeführt sind, sind mit der Ringantenne über ein Datenkabel verbunden. Diese restliche Empfangseinheit kann beispielsweise am Gürtel getragen oder auf einem Labortisch aufgestellt werden. Diese Trennung der Ringantenne von der sonstigen Empfangseinheit verringert die Baugröße und das Gewicht der am Kopf des Patienten aufzusetzenden Komponente.

Das Gehäuse der Ringantenne dieser nicht weiter dargestellten Ausführung kann charakteristische Ausbuchtungen und/oder Mulden und/oder sonstige Vertiefungen aufweisen, die beim manuellen Aufsetzen der Ringantenne zur Erleichterung der Positionierung von dieser dienen. Es können beispielsweise drei Ausbuchtungen und/oder Mulden und/oder Vertiefungen vorliegen, die in etwa gleich verteilt am Außenumfang der Ringantenne angeordnet sind.

Das Gehäuse der Ringantenne kann radial verlaufende Vertiefungen an der vom Patientenkopf in der aufgesetzten Stellung abgewandten Oberseite der Ringantenne aufweisen. Diese radial verlaufenden Vertiefungen können als Befestigungshilfen für ein Klettband zur Fixierung der Ringantenne an der Kopfhaube 14 dienen.

## Patentansprüche

1. Verfahren zur Übertragung von Sensordaten eines implantierbaren Hirnparametersensors auf ein externes Datenlesemodul (18) mit folgenden Schritten:
- Senden eines von einer Hochfrequenzquelle des Datenlesemoduls (18) erzeugten Hochfrequenz-Signals,
- Empfangen des Hochfrequenz-Signals mit einer Sendeeinheit (4) des Hirnparametersensors,
- Gleichrichten des Hochfrequenz-Signals,
- Aufladen eines Kondensators (6) zur zeitweiligen Energieversorgung der Sendeeinheit (4),
- Deaktivieren des Hochfrequenz-Signals nach dem Ladevorgang,
- nach dem Deaktivieren Aufnehmen eines Hirnparameter-Messsignals, insbesondere eines Hirndruckwertes, wobei während des Aufnehmens des Hirnparameter-Messsignals eine Energieversorgung des Hirnparametersensors über den Kondensator (6) erfolgt,
- Aktivieren des Hochfrequenz-Signals nach dem Messsignal-Aufnehmen,
- Senden des Hirnparameter-Messsignals mit dem Hochfrequenz-Signal als Trägerfrequenz mittels der Sendeeinheit (4) an eine Empfangseinheit (8) des Datenlesemoduls (18).

2. Verfahren nach Anspruch 1, **dadurch gekennzeichnet, dass** das Hirnparameter-Messsignal als digital moduliertes Signal an die Empfangseinheit (8) gesendet wird.

3. Verfahren nach Anspruch 2, **dadurch gekennzeichnet, dass** das Digitalisieren des Hirnparameter-Messsignals mittels eines A/D-Wandlers erfolgt.

4. Verfahren nach einem der vorstehenden Ansprüche, **gekennzeichnet durch** ein Überführen des modulierten Hochfrequenz-Signals in ein digitales, insbesondere PC-kompatibles, Signal in der Empfangseinheit (8).

5. Verfahren nach Anspruch 4, **dadurch gekennzeichnet, dass** das PCkompatible Signal ein USB- oder RS232-Signal ist.

6. Verfahren nach Anspruch 4 oder 5, **dadurch gekennzeichnet, dass** das überführte Messsignal über ein Signalkabel (17) an das Datenlesemodul (18) übertragen wird.

7. Verfahren nach Anspruch 6, **dadurch gekennzeichnet, dass** das übertragene Signal über ein weiteres Signalkabel (19) an eine Datenverarbeitungs- und Darstellungseinrichtung (20) zur weiteren Verarbeitung und Darstellung übermittelt wird.

8. Verfahren nach einem der vorstehenden Ansprüche, **gekennzeichnet durch** ein regelmäßiges Wiederholen aller Verfahrensschritte.

9. Verfahren nach Anspruch 8, **dadurch gekennzeichnet, dass** das regelmäßige Wiederholen aller Verfahrensschritte über einen längeren Zeitraum, insbesondere im Verlauf mehrerer Tage, erfolgt.
